# EUROPEAN PATENT APPLICATION

(11) **EP 4 583 117 A1**
(43) Date of publication of application: **09.07.2025**
(21) Application number: 24150531.2
(22) Date of filing: 05.01.2024
(51) Int. Cl.: G16H 20/40, G16H 30/40, G16H 40/63, G16H 50/20, G16H 15/00

(54) **METHODS AND DEVICES FOR SUPPORTING A MEDICAL PROCEDURE**

(71) Applicant: Leica Microsystems NC, Inc., Durham, NC 27703 (US)
(72) Inventor: Maione, Bryan, Durham, 27703 (US)
(74) Representative: J A Kemp LLP

(57) **Abstract**

A first aspect of the present disclosure is related to a method for supporting a medical procedure,
comprising the steps:
- obtaining medical information from a medical procedure with a measurement system yielding medical measurement data;
- providing the medical measurement data to a first machine learning system yielding medical-related textual data;
- storing the medical-related textual data for later use.

## Description

### Technical Field

This disclosure is related to computer-implemented methods for supporting a medical procedure and to systems for supporting a medical procedure.

### Background

In medical assessments and surgeries, summarizing medical procedures and documenting outcomes, findings, and any observed complications is an essential aspect of maintaining accurate medical records and ensuring patient safety and continuity of care. Surgeons rely on detailed surgical summaries to provide comprehensive post-operative care and to make informed medical assessments during and after medical procedures. Additionally, medical practitioners require detailed preoperative and intraoperative information, including patient history, diagnostic tests, imaging, and intraoperative findings, medical opinions, and related scientific information. Combining this to reduce cognitive load of a medical practitioner and to enable fast and high-quality decisions is a critical task. Improvements in this field are desirable.

### Summary

An object of the present disclosure is to improve an execution of a medical procedure.

This object is solved by the disclosed embodiments, which are defined in particular by the subject matter of the independent claims. The dependent claims provide information for further embodiments. Various aspects and embodiments of these aspects are also disclosed in the summary and description below, which provide additional features and advantages.

A first aspect of the present disclosure is related to a method for supporting a medical procedure,
comprising the steps:
- obtaining medical information from a medical procedure with a measurement system yielding medical measurement data;
- providing the medical measurement data to a first machine learning system yielding medical-related textual data;
- storing the medical-related textual data for later use.

An obtaining of an information, such as medical information, in the sense of the present disclosure can comprise receiving and/or a fetching of this information. Additionally or alternatively, this can comprise determining this information based on other received/fetched information.

Medical information in the sense of the present disclosure can comprise information from an examination and/or an operation. Additionally or alternatively, medical information can comprise information before and/or after an examination and/or operation.

A measurement system in the sense of the present disclosure can comprise one or more sensors configured to observe information generated during a medical assessment/examination and/or during a surgery. Therefore, the measurement system observes the place in which the assessment/examination or the surgery takes place. A measurement system can comprise a plurality of sensors that are configured to observe the medical environment and/or the medical procedure, e.g., a camera and an electrocardiogram, and/or information from systems that are used to perform the medical procedure. Medical measurement data is any data directly measured from the measurement system. Additionally the data can be filtered or transformed for further processing by the first machine learning system.

A first machine learning system in the sense of the present disclosure can be any machine learning system to generate medical-related textual data from medical measurement data. A machine learning system designed to generate text from non-textual input data can comprise one or more methods for multimodal learning. Thereby, medical measurement data from different modalities, e.g. images, audio, or video, are processed to generate relevant textual output. In case of visual medical measurement data, the first machine learning system comprises computer vision techniques to understand the content of the visual medical measurement data. For audio medical measurement data, the first machine learning system comprises speech recognition and/or audio analysis. Based on the analysis's features are extracted from the medical measurement data. In the case of images, this can comprise identifying objects, colors, and/or spatial relationships. For audio, it might comprise extracting speech features, tone, and/or background noises. The extracted features can then be fed into a language model, e.g. a large language model like a transformer-based model, such as GPT. The language model can be trained to generate human language. The language model can use the extracted features from the medical measurement data to provide a contextualized and/or transcribed output.

A machine learning system for the embodiments of the present disclosure can comprise a natural language processing system, in particular combined with and/or based on a neural network. A m machine learning system can be based on supervised or unsupervised learning, reinforcement learning, transformer models and/or LSTM-models. A convolutional neural network can comprise a standard neural network, e.g. a VGG and/or a ResNET.

A providing of information in the sense of the present disclosure, such as medical measurement data and/or medical-related textual data, can comprise sending the information and/or storing the information for others to fetch, e.g. storing the information in a shared memory.

An embodiment of the present disclosure is related to a method for supporting a medical procedure,
wherein medical information comprises one or more of the following:
- dictational information;
- conversational information;
- information from a patient;
- environmental information of the medical procedure.

Medical information can comprise information from medical records, allergies, medications, and any pre-existing conditions. Additionally or alternatively, medical information can comprise surgical plan and procedure details. This can comprise specific steps of a surgery and/or description of one or more instruments required. Medical information can comprise anesthesia, e.g. a dosage and/or the patient's response to anesthesia. Additionally or alternatively, medical information can comprise any data from a vital signs monitoring and/or any other devices that observe medical-related information, e.g., a heart rate, a blood pressure, an oxygen saturation, and/or a respiratory rate. Additionally or alternatively, medical data can comprise diagnostic data, e.g., X-rays, CT scans, or MRIs, and/or any intraoperative data obtained, e.g., to guide a surgical process. Additionally or alternatively, medical data can comprise sterilization and instrument status information. Additionally or alternatively, medical data can comprise communication or dictation of medical staff related to pre-operative briefing, intraoperative updates, requests for instruments or assistance, documentation, postoperative debriefing, and/ or handoff communications, e.g. if the patient is transferred to another location and/or another medical personnel.

An embodiment of the present disclosure is related to a method for supporting a medical procedure,
wherein medical information comprises an augmented reality information.

Medical augmented reality information can refer to information generated by the use of augmented reality technology for a medical procedure, such as a surgery. Augmented reality technology overlays digital information (like images, data, and text) onto the real-world environment, often through devices like AR glasses, headsets, or smartphone apps. Medical augmented reality information is in particular provided in medical procedures to enhance the visualization of complex medical information. Medical augmented reality information can be provided directly from the augmented reality technology, i.e. before its overlay to a medical procedure, to a first machine learning system. For example, medical augmented reality data for display on a screen and/or on surgery glasses can be provided to the first machine learning system from the augmented reality device directly. Additionally or alternatively, this kind of data can be observed, e.g. by a camera that records a screen with medical augmented reality data, and the observed/measured medical augmented reality information is provided (as medical measurement data) to the first machine learning system.

An embodiment of the present disclosure is related to a method for supporting a medical procedure,
wherein the measurement system used to capture the medical procedure comprises one or more of the following devices:
- a camera;
- a microphone;
- a laparoscope;
- a medical sensor;
- a medical device
- a microscope.

A camera and/or a microphone can be mounted, e.g., in an operating room to measure the signals from a patient, a surgery, and/or surgical personnel. A camera (and also a microphone) can also be a medical device used in the surgery (or any other medical procedure). For example, the camera can be a laparoscopic camera or a microscope. Medical measurement information can come from any device used during a medical procedure. Therefore, such a device does not necessarily be configured to produce data for a method according to the first aspect of the present disclosure. Data from a device can be taken as "raw-data" and, if necessary, can be transformed into medical measurement data suitable for a first machine learning system or for further steps within a method according to the first aspect.

Devices that generate information that can be used as medical information can comprise, e.g., an electrocardiogram (ECG or EKG), a pulse oximeter, a capnography machine, a blood pressure monitor, an anesthesia machine, an intraoperative monitoring equipment, an intracranial pressure monitor, a temperature monitor, surgical navigation systems, and/or devices that administer fluids, such as medications or nutrients, intravenously or epidurally, and provide information on the rate and volume of delivery.

Additionally or alternatively, devices that do not explicitly configured to generate a user information can also be used as medical information providers. Such devices can comprise, e.g., infusion pumps, cauterization devices, electric scissors, a suction device, etc., which in particular report their settings and/or states in real time. The medical information of these devices can be e.g. an infusion rate, an infusion amount, a cauterization current, a scissors-force (measured e.g. as current).

An embodiment of the present disclosure is related to a method for supporting a medical procedure,
wherein the first machine learning system is trained with information from prior medical procedures.

The first machine learning system can in particular be trained with speech, audio, and/or image information obtained from medical procedures that were of the same type of medical procedure for which a method according to the first aspect of the present disclosure is to be used for.

An embodiment of the present disclosure is related to a method for supporting a medical procedure,
wherein the medical-related information is provided as a summary of the obtained medical measurement data.

A medical measurement data can comprise information from different modalities (vision, audio, text). Medical measurement data can be preprocessed in order to be integrated with each other. Text data can be extracted from transcripts, articles, or captions. Image and video data can be processed to identify key frames, objects, and/or medical relevant content. Audio information can be transcribed and/or analyzed with respect to tone, emotion, or/or background sounds for additional context.

Afterwards a features extraction can be performed. For textual measurement data this can be performed based on natural language processing techniques for textual data to identify keywords, topics, or/or sentiments. For visual/image data a processing can be performed to interpret images and video frames and identify important medical-related elements. For audio information, audio processing techniques can be performed to understand spoken language, tone, and other auditory cues that related to a desired medical context.

Based on multimodal information, an in particular on extracted features from a multi-model medical information, a cross-modal correlation can be performed to integrate information from different modalities and in particular to determine one or more relationships between data from different modalities. For example, textual and/or audio descriptions can be aligned with images and/or video clips. Additionally or alternatively audio cues can be correlated with visual data. Therefore, a content of a measurement information, in particular a content of an extracted feature can be identified. Based on the identified content, two or more medical informations from different modalities can be related to each other. For example, a video sequence that shows the cauterization of a blood vessel can be identified by the first machine learning system, to be a 'cauterization of a blood vessel'. Additionally, an audio information from the surgeon can comprise information on the reason for the cauterization of the blood vessel. The first machine learning system can also qualify this audio information as 'cauterization of a blood vessel'. Both informations can then be integrated with each other. Other information that seems not to bear relevant content can be left out. Other information can be used to complement an alignment, e.g. time information. Information from different modalities that are measured on the same time or at least at overlapping time phases, are more likely to comprise information related to the same topic.

In an alternative implementation of this embodiment, a separate summarization machine learning system can be used for providing a summarized medical-related textual information. The summarization machine learning system can be trained specially for summarizing medical information presented in a textual form.

An embodiment of the present disclosure is related to a method for supporting a medical procedure,
comprising the step:
- provide medical measurement data to the summarized medical-related textual data.

A summarized medical-related textual data can be a text complemented with visual and/or audio elements that support or enhance the textual information. Additionally or alternatively, a medical-related textual data can be reduced or summarized to obtain a reduced/summarized medical-related textual data.

An embodiment of the present disclosure is related to a method for supporting a medical procedure,
comprising the steps:
- providing the medical-related textual information to an assistance machine learning system configured to provide assistance medical information based on the medical-related textual information;
- providing the assistance medical information to a user.

An assistance machine learning system in the sense of the present disclosure can be any machine learning system that is configured to process textual information. This can in particular be a large language model and/or a transformer-based neural network model. The assistance machine learning model can generate text and/or audio information as assistance medical information. Furthermore, the assistance machine learning system can be configured to generate multimodal information to be presented as assistance medical information. For example, a specific text can be presented together with a specific image or image sequence as a multimodal assistance information.

An embodiment of the present disclosure is related to a method for supporting a medical procedure,
comprising the step:
- providing the medical measurement data to the assistance machine learning system additional to the medical-related textual information.

The assistance machine learning system can extract and/or select the medical measurement data to be presented together with the medical-related textual information. The selection can be based on the alignment mechanisms described above for the embodiments related to summarization. Additionally or alternatively, medical measurement data from prior medical procedures, e.g. prior surgeries, can be presented together with the medical-related textual information.

An embodiment of the present disclosure is related to a method for supporting a medical procedure,
comprising the step:
- training the assistance machine learning system with medical information, in particular based on medical measurement data and/or medical-related textual information of prior medical procedures.

The training of the assistance machine learning system can in particular be focused on retrieving additional information, e.g. from a data source such as the Internet/WWW, that can be aligned or used in combination with a medical measurement information and/or a medical-related textual information. In particular a training can be focused on difficulties and/or aspect that are not comprised in an obtained medical measurement information and or a computed medical-related textual information. In this way, the assistance medical system can comprise complementary data for a medical personnel.

An embodiment of the present disclosure is related to a method for supporting a medical procedure,
comprising the steps:
- providing a context information to the assistance machine learning system;
- providing the assistance medical information in view of the context information to the user.

Context information can be e.g. a scientific paper, a specific information from a prior surgery, a textual and/or auditive information provided by a medical personnel. By using context information, the assistance medical system can be directed to providing assistance related to the provided context information. This can improve a quality of the provided assistance information, i.e. a usefulness of the assistance information in a specific medical situation. For example, a surgeon can provide a medical scientific paper that deals with a technique to put together two parts of a broken bone. When provided with this textual information, the assistance machine learning system can evaluate the obtained medical measurement data and/or the obtained medical-related textual data in view of the provided context information and provide assistance information accordingly.

An embodiment of the present disclosure is related to a method for supporting a medical procedure,
comprising the steps:
- obtaining a context information based on the medical measurement data and/or the medical-related textual data;
- providing the context information to the assistance machine learning system;
- providing a further assistance information based on the context information to a user.

Context information can be obtained automatically based on an obtained medical measurement date and/or a computed medical-related textual data.

The latter can be, e.g., a summarized medical-related textual data and/or features extracted from one of these obtained/computed data sets. For example, a computed medical-related textual data is focused on a broken thigh bone. This can be, e.g., a statistically measurable number of instances in the medical-related textual data which mention a broken thigh bone. Based on this information, the assistance machine learning system can retrieve information on surgical operation of broken thigh bones, or even on a more specific topic in this context and evaluate the obtained medical measurement information and/or the medical-related textual information, in this view and provide assistance information accordingly.

An embodiment of the present disclosure is related to a method for supporting a medical procedure,
comprising the steps:
- obtaining from a user a context evaluation information;
- providing the assistance information and/or the further assistance information based on the context information and on the context evaluation information.

A user can provide context evaluation information, e.g., based on a prompting question, which the user provides in text form and/or in audio form. Additionally or alternatively, user context information can be provided by a selection of a part in an image of a medical device, or any other device used for observing a medical procedure. For example, a surgeon can select, e.g. by a computer mouse, in an image from a laparoscopic camera a certain part of a blood vessel. The image can then be used to retrieve additional context related assistance information. Therefore the assistance machine learning system can be, e.g., a convolutional neuronal network that processes the obtained user context evaluation information together with a medical-related textual information and/or a medical measurement information to obtain an appropriate assistance information.

An embodiment of the present disclosure is related to a method for supporting a medical procedure,
wherein the context evaluation information is captured during the medical procedure, in particular by the measurement system that is configured to obtain the medical information.

For example, the context evaluation information can be a feature of a knee joint captured by a laparoscopic camera. The features is selected by a surgeon on a touch screen placed adjacent to a surgery table.

A second aspect of the present disclosure is related to a system for supporting a medical procedure
configured to:
- obtain medical information from a medical procedure with a measurement system yielding medical measurement data;
- provide the medical measurement data to a first machine learning system yielding medical-related textual data;
- store the medical-related textual data for later use.

A system can be implemented to execute a method according the first aspect of the present disclosure.

### Brief description of the figures

Further advantages and features result from the following embodiments, some of which refer to the figures. The figures do not always show the embodiments to scale. The dimensions of the various features may be enlarged or reduced, in particular for clarity of description. For this purpose the figures are at least partially schematized.
Fig. 1 illustrates a method supporting a medical procedure according to an embodiment of the present disclosure.
Fig. 2 illustrates a set-up for supporting a surgical procedure according to an embodiment of the present disclosure.
Fig. 3 illustrates a microscope arrangement used for a system according to an embodiment of the present disclosure.

In the following description reference is made to the accompanying figures which form part of the disclosure, and which illustrate specific aspects in which the present disclosure can be understood. Identical reference signs refer to identical or at least functionally or structurally similar features.

In general, a disclosure of a described method also applies to a corresponding device (or apparatus) for carrying out the method or a corresponding system comprising one or more devices and vice versa. For example, if a specific method step is described, a corresponding device may include a feature to perform the described method step, even if that feature is not explicitly described or represented in the figure. On the other hand, if, for example, a specific device is described on the basis of functional units, a corresponding method may include one or more steps to perform the described functionality, even if such steps are not explicitly described or represented in the figures. Similarly, a system can be provided with corresponding device features or with features to perform a particular method step. The features of the various exemplary aspects and embodiments described above or below may be combined unless expressly stated otherwise.

### Detailed description

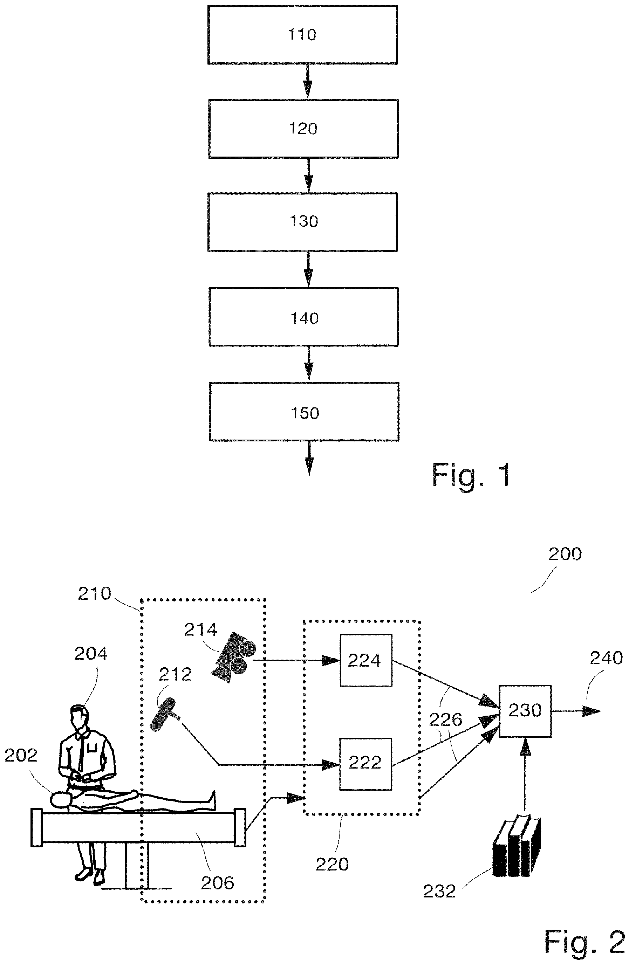

Fig. 1 illustrates a flow chart 100 for a method supporting a medical procedure according to an embodiment of the present disclosure. In a first step 110, a medical information from a medical procedure is captured with a measurement system yielding medical measurement data. The medical measurement data can be unimodal or multimodal, e.g. comprising visual, audio, and text data.

In a second step 120, the medical measurement data is provided to a first machine learning system yielding medical-related textual data. This is textual data is describing the situation represented by the measured and multimodally integrated medical-related textual data. The medical-related textual data can be presented as a textualization of everything that can be inferred from the medical measurement data. Alternatively, the medical-related data is computed as a summarized data that focusses on key aspects of the information comprised in the measured medical data.

In a third step 130, non-textual data measured during the medical procedure is aligned to the medical-related textual data. For example, a video sequence is added to a certain medical-related textual data item. Thereby, the information of the medical-related textual data is improved, and understandability of the data is increased.

In a fourth step 140, medical-related textual data is stored for further use. This further use can be e.g. a use for an assistance machine learning system.

In fifth step 150 the medical-related textual information is provided to an assistance machine learning system. The assistance machine learning system is configured to provide assistance medical information based on the medical-related textual information. Assistance medical information can be information retrieved from the Internet/WWW or from a digital library and that is selected on the basis of the medical related textual information and/or on the medical measurement information. Additionally or alternatively, a context information, e.g. a scientific paper, is provided to the assistance machine learning system and the system evaluates the medical-related textual information and/or the medical measurement information in view of the provided context information and afterwards assists a user with the evaluation result.

Fig. 2 illustrates a setup 200 for supporting a surgical procedure according to an embodiment of the present disclosure. In the setup, a patient 202 lies on an operation table 206 and a surgeon 204 performs a surgery on the patient.

Different devices 210 in the operating room provide medical information from the surgery and its environment. A microphone 212 captures audio signals from the operating room, including the speech of the surgeon and other medical personnel. A video camera 214 captures video information from the surgery. Furthermore, a video camera from surgical device, e.g., a laparoscopic camera (not depicted), can be used to capture medical information. Furthermore, vital signs monitoring equipment 206 can capture medical information.

The captured medical information, i.e. the medical measurement information, is then provided to a first machine learning system 220. The first machine learning system can comprise one or more machine learning subsystems that provide textual information that represent the situation in the operation room. The speech information can be textualized by a first subsystem 222. This subsystem can be trained in particular with the voices of the personnel performing the surgery. Additionally or alternatively, the subsystem 222 can be trained with medical information, in particular of the conducted surgery type. The video information is transformed to text by the subsystem 224. The video captioning system 224 Video Captioning" can comprise combination of computer vision and natural language processing techniques to convert the visual content of a video into a textual description or transcript. In order to capture temporal aspects of the video, recurrent neural networks, long shortterm memory (LSTM) networks and/or transformer-networks can be employed. Once the visual features and temporal information are extracted from the video, the system can use NLP techniques to generate descriptive text. This could comprise recurrent models like LSTMs or transformer-based models like generative pretrained transformers. The textualized information 226 can be the full information or already focused information, i.e. a summary of the surgery.

The textualized information is then provided to an assistance machine learning system 230. The assistance machine learning system comprises a large language model, i.e. based on a generative pre-trained transformer. The assistance machine learning system 230 can be provided with a specific context information to provide a retrieval-augmented output generation. In this way the vast knowledge of a large language model can be focused on the specific context 232, provided e.g. by a book or a scientific paper. The output 240 can further be provided as speech in order to provide the information effectively to a user, e.g. during the medical procedure. The described embodiment can aid in the detection and management of surgical complications and can help reducing stress in the operating room and potentially improving an outcome of a medical procedure.

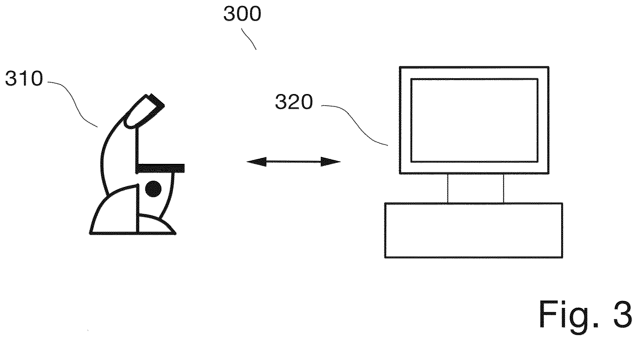

A microscope may be part of or connected to a system as described in connection with one or more of the Figs. 1 and 2 or further above. Fig. 3 shows a schematic illustration of a system 300 configured to perform a method described herein. The system 300 comprises a microscope 310 and a computer system 320. The microscope 310 is configured to take images and is connected to the computer system 320. The computer system 320 is configured to execute at least a part of a method described herein. The computer system 320 may be configured to execute a machine learning algorithm. The computer system 320 and microscope 310 may be separate entities but can also be integrated together in one common housing. The computer system 320 may be part of a central processing system of the microscope 310 and/or the computer system 320 may be part of a subcomponent of the microscope 310, such as a sensor, an actor, a camera, or an illumination unit, etc. of the microscope 310.

The computer system 320 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 320 may comprise any circuit or combination of circuits. In one embodiment, the computer system 320 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 320 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 320 may include one or more storage devices, which may include one or more memory elements suitable to the particular application, such as a main memory in the form of random-access memory (RAM), one or more hard drives, and/or one or more drives that handle removable media such as compact disks (CD), flash memory cards, digital video disk (DVD), and the like. The computer system 320 may also include a display device, one or more speakers, and a keyboard and/or controller, which can include a mouse, trackball, touch screen, voice-recognition device, or any other device that permits a system user to input information into and receive information from the computer system 320.

Some or all of the method steps may be executed by (or using) a hardware apparatus, like for example, a processor, a microprocessor, a programmable computer, or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine-readable carrier.

Other embodiments comprise the computer program for performing one of the methods described herein, stored on a machine-readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or nontransitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device, or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied, and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate, or apply the knowledge.

Machine-learning algorithms are usually based on a machine-learning model. In other words, the term "machine-learning algorithm" may denote a set of instructions that may be used to create, train, or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of reference signs

- 100: Method flow chart
- 110: obtaining medical information
- 120: computing medical related textual data
- 130: add multimodal non textual data
- 140: storing the medical related textual data
- 150: computation of assistance information
- 200: surgical setup
- 202: patient
- 204: surgical personnel
- 206: vital signs monitoring
- 210: sensors
- 212: microphone
- 214: video camera
- 220: first machine learning system
- 222: speech2text system
- 224: video2text system
- 226: textualized information
- 230: assistance machine learning system
- 232: specific context for evaluation
- 240: output
- 300: microscope system
- 310: microscope
- 320: computer

## Claims

1. A computer-implemented method for supporting a medical procedure, comprising the steps:
- obtaining medical information from a medical procedure with a measurement system (210) yielding medical measurement data;
- providing the medical measurement data to a first machine learning system (220) yielding medical-related textual data (226);
- storing the medical-related textual data for later use.

2. The method according to the preceding claim,
wherein medical information comprises one or more of the following:
- dictational information;
- conversational information;
- information from a patient;
- environmental information of the medical procedure.

3. The method according to the preceding claim,
wherein medical information comprises an augmented reality information.

4. The method according to the preceding claim,
wherein the measurement system used to capture the medical procedure comprises one or more of the following devices:
- a camera (214);
- a microphone (212);
- a laparoscope;
- a medical sensor;
- a medical device;
- a microscope (310).

5. The method according to one of the preceding claims,
wherein the first machine learning system (220) is trained with information from prior medical procedures.

6. The method according to one of the preceding claims,
wherein the medical-related information is provided as a summary (226, 240) of the obtained medical measurement data.

7. The method according to the preceding claim,
comprising the step:
- provide medical measurement data to the summarized medical-related textual data.

8. The method according to one of the preceding claims,
comprising the steps:
- providing the medical-related textual information to an assistance machine learning system (230) configured to provide assistance medical information (240) based on the medical-related textual information;
- providing the assistance medical information to a user.

9. The method according to the preceding claim,
comprising the step:
- providing the medical measurement data to the assistance machine learning system (230) additional to the medical-related textual information.

10. The method according to one of the preceding claims 8 or 9,
comprising the step:
- training the assistance machine learning system (230) with medical information, in particular based on medical measurement data and/or medical-related textual information (226) of prior medical procedures.

11. The method according to one of the preceding claims 8-10,
comprising the steps:
- providing a context information (232) to the assistance machine learning system (230);
- providing the assistance medical information in view of the context information to the user.

12. The method according to the preceding claims 8-11,
comprising the steps:
- obtaining a context information (232) based on the medical measurement data and/or the medical-related textual data (226);
- providing the context information to the assistance machine learning system (230);
- providing a further assistance information based on the context information to a user.

13. The method according to the preceding claims 8-12,
comprising the steps:
- obtaining from a user a context evaluation information (232);
- providing the assistance information and/or the further assistance information based on the context information and on the context evaluation information.

14. The method according to the preceding claim,
wherein the context evaluation information is captured during the medical procedure, in particular by the measurement system (220) that is configured to obtain the medical information.

15. A system (300) for supporting a medical procedure
configured to:
- obtain medical information from a medical procedure with a measurement system (310) yielding medical measurement data;
- provide the medical measurement data to a first machine learning system yielding medical-related textual data;
- store the medical-related textual data for later use.
